# EUROPEAN PATENT SPECIFICATION

(11) **EP 3 847 990 B1**
(45) Date of publication and mention of the grant of the patent: **06.04.2022**
(21) Application number: 20151338.9
(22) Date of filing: 13.01.2020
(51) Int. Cl.: A61B 90/00, A61B 34/20, A61B 34/10

(54) **TECHNIQUE OF CONTROLLING DISPLAY OF A NAVIGATION VIEW INDICATING AN INSTANTANEOUSLY CHANGING RECOMMENDED ENTRY POINT**
TECHNIK ZUR STEUERUNG DER ANZEIGE EINER NAVIGATIONSANSICHT, DIE EINEN SICH AUGENBLICKLICH ÄNDERNDEN EMPFOHLENEN EINSTIEGSPUNKT ANZEIGT
TECHNIQUE DE COMMANDE D'AFFICHAGE D'UNE VUE DE NAVIGATION INDIQUANT UN POINT D'ENTRÉE RECOMMANDÉ CHANGEANT INSTANTANÉMENT

(43) Date of publication of application: 14.07.2021
(73) Proprietor: Stryker European Operations Limited, Carrigtwohill, Co. Cork T45 HX08 (IE)
(72) Inventor: WAPLER, Matthias, 79199 Kirchzarten (DE)
(74) Representative: Röthinger, Rainer

(56) References cited:
- US-A1- 2008 221 520
- US-A1- 2018 217 734
- US-B1- 6 529 765

## Description

### Technical Field

The present disclosure generally relates to surgical navigation, namely to a computer-implemented method for controlling a navigation view for surgical navigation. A processor, a computer-readable medium and a data carrier signal are also disclosed.

### Background

During surgical navigation, a surgeon may use a surgical navigation system to help align a surgical instrument relative to a patient. To this end, positions and orientations of surgical objects, such as the surgical instrument and the patient, can be determined as a basis for providing navigation instructions to the surgeon.

In an exemplary application of a surgical navigation system, the surgical instrument and the patient are each associated with a tracking marker, wherein three dimensional image data of the patient previously obtained by, for example, a computer tomography (CT) scan is registered with the patient tracking marker and the surgical instrument is registered with the instrument tracking marker. By tracking the tracking markers of the patient and the surgical instrument, the position and orientation of both the patient and the surgical instrument, and thus a spatial relationship between the three dimensional image data of the patient and the position and orientation of the surgical instrument can be determined.

In several surgical procedures surgeons need to align a surgical instrument such as a shunt stylet, drill, awl, tap, screw-driver, biopsy needle, etc., relative to the patient's body free-handedly, in order to align the surgical instrument with a pre-operatively planned trajectory. A pre-operatively planned trajectory (i.e., planned trajectory) is typically defined by a planned entry point and a planned target. The entry point typically defines a point where an incision shall be made on the skin of the patient's body or where an opening shall be drilled in the patient's skull, and through which the surgical instrument shall enter the patient's body. The entry point can define the point on the bony surface of a vertebra where an implant such as a pedicle screw shall be inserted. The target and the planned entry point can be defined in the three dimensional image data of the patient. Examples of such targets are a target point (i.e., a point in ventricles), a tumor, an anatomical organ to be treated, desired tip position of a pedicle screw and more. The target may be defined by defining a point or outlining a geometry in the three dimensional image data.

After determining planning data describing the planned entry point and the target, surgical navigation may be performed during which the aforementioned determined spatial relationship is, for example, displayed on a screen in form of a navigation view, helping the surgeon guide the surgical instrument relative to the patient.

In some cases, the surgeon may decide during surgical navigation that a slightly different entry point compared with the planned entry point is preferable. In other cases, the surgeon may not be able to perfectly align the surgical instrument to the planned entry point.

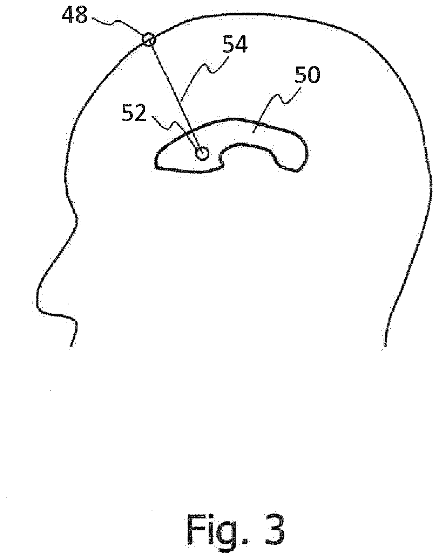

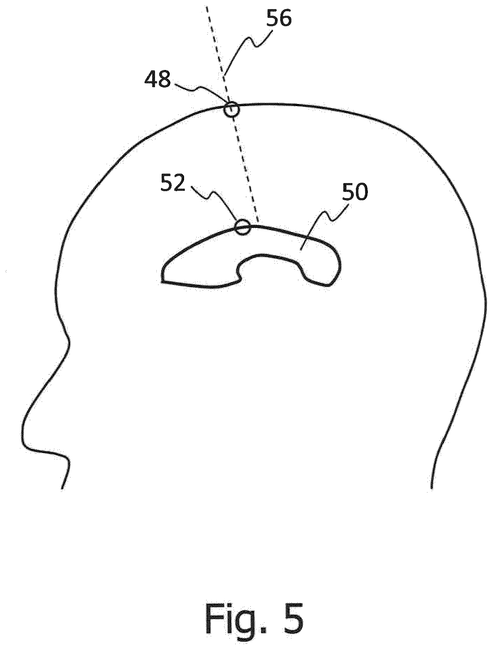

Some navigation views may support two different alignment modes. In a first alignment mode, the surgical instrument is aligned with both the planned entry point and the planned trajectory. If the trajectory is perfectly aligned, the outcome equals that shown in Fig. 3. However, in practice the typical outcome corresponds to that shown in 6. In a second alignment mode, the surgical instrument is aligned with the target only. The trajectory of the surgical instrument may then pass exactly through the target but may miss the planned entry point, as shown in Fig. 5.

The prior art does not provide a fast and accurate alignment of navigated surgical instruments in order to accurately and reliably reach a planned target.

Document US6529765 B1 describes a method and apparatus for stereotactic surgery on a body, such as surgery on the brain or spine. A cranial probe is used to determine an actual entry point. A computer display shows a composite image, which includes a three-dimensional surface view and three planar views of a navigational view determined by the planned entry and target points. The surgical trajectory is optionally replanned to go through the centre of an actual mounted position of a guidance fixture, rather than a planned entry point.

### Summary

There is a need for a computer-implemented method of controlling display of a navigation view that solves one or more of the aforementioned or other problems.

According to a first aspect, a computer-implemented method of controlling display of a navigation view for surgical navigation is provided. The method comprises obtaining planning data defining a planned entry point and a planned target, wherein the planned entry point and the planned target are defined in a coordinate system of a computer model of at least one anatomical element. The method further comprises obtaining tracking data describing a current pose of a surgical instrument in the coordinate system and determining, based on at least the tracking data, a current entry point lying on a trajectory having a fixed spatial relationship with the surgical instrument. The method further comprises controlling display of a navigation view visualizing at least a recommended entry point, wherein the recommended entry point is instantaneously changed from the planned entry point to the current entry point, upon a distance between the planned entry point and the current entry point fulfilling a predefined condition.

The planned entry point for example fulfills at least one condition chosen from being distant from the planned target, being not comprised in the planned target and being not identical to the planned target. The planned entry point may lie on, a predetermined distance below or a predetermined distance above an outer surface of the computer model of the at least one anatomical element, for example in case the computer model is determined based on computer tomography, CT, patient image data. In one example, the planned entry point lies on the outer surface of the computer model, whilst the planned target lies beneath the outer surface of the computer model. The outer surface may correspond to a bony surface of the anatomical element.

The at least one anatomical element is for example an anatomical organ, a bone, a limb or else. In one example, the at least one anatomical element comprises all body parts within a section of or within the whole patient's body. The section may be limited in size depending on the imaging technology used. In other words, the at least one anatomical element may comprise all anatomical elements described by patient image data. The patient image data is for example obtained by medical image acquisition of the patient and may in particular be CT patient image data or magnetic resonance, MR, patient image data. The at least one anatomical element may essentially consist of a certain tissue type such as bone, muscle, skin or tendon. For example, the at least one anatomical element has a predetermined transparency for X-rays, resulting in a predetermined range of Hounsfield values in X-ray or CT images.

The planned target for example is equal to or comprises a target point or an outline of an extended anatomical target. The planned target may be a volume in the computer model, for example a volume comprising or describing an anatomical element such as an anatomical organ, a tumor, a region of interest or else.

The planning data may be obtained by obtaining first data describing the planned entry point and obtaining second data describing the target. The orientation of the surgical instrument in the coordinate system may be obtained by obtaining an orientation of the surgical instrument in a tracking coordinate system and transforming this orientation into the coordinate system based on a known transformation between the tracking coordinate system and the coordinate system.

The trajectory is for example (i.e., essentially) parallel to a longitudinal axis of the surgical instrument, (i.e., essentially) parallel to an insertion direction of the surgical instrument or (i.e., essentially) orthogonal to a handle of the surgical instrument.

The navigation view for example visualizes the recommended entry point by aligning or orienting the navigation view with respect to the recommended entry point.

The predefined condition is for example that the distance between the planned entry point and the current entry point is lower than a threshold. The threshold may be a predetermined value such as for example 0.5mm, 1mm, 1.5mm, 3 mm or 4 mm, in particular 2mm. The threshold in one example is larger than 0mm, in particular larger than 1mm or larger than 2mm. The threshold may be obtained in a previous method step. The threshold may be set by a user. For example, the threshold is a value depending on a spatial relationship between the planned entry point and the target. In this case, the threshold may correspond to a certain percentage of the distance between the planned entry point and the target. The threshold is for example a value depending on a spatial relationship between the surgical instrument and at least one of the target, the current entry point and the planned entry point. The threshold may be chosen such that the instantaneous change of the recommended entry point can be detected by a user solely from the navigation view. The threshold may be larger than a distance described by a single pixel of the navigation view. For example, the threshold is larger than a predetermined percentage of a width of a shaft of the surgical instrument.

The navigation view may visualize the recommended entry point and the planned target. The navigation view for example visualizes the recommended entry point and the planned target by displaying the recommended entry point and the target on a representation of the computer model and/or by orienting the navigation view with respect to at least one of the recommended entry point and the target.

The navigation view in one example has a viewing direction corresponding to a straight line between the recommended entry point and the planned target.

The navigation view may be a bullseye view centered on a straight line between the recommended entry point and the planned target. In another variant, the navigation view comprises a bullseye centered on the straight line between the recommended entry point and the planned target. The bullseye or the bullseye view may comprise at least one concentric line centered on the straight line between the recommended entry point and the planned target. The at least one concentric line may be a two-dimensional line lying in a plane, which plane is orthogonal to the straight line between the recommended entry point and the planned target. The bullseye or the bullseye view for example has the straight line between the recommended entry point and the planned target as a viewing direction. In addition, or as an alternative, to the bullseye, the navigation view may comprise one or more crosshairs, for example a first crosshair essentially orthogonal to a second crosshair. The one or more crosshairs may intersect the straight line between the recommended entry point and the planned target.

The navigation view in one variant comprises a visualization of a relative spatial relationship between the recommended entry point and the planned target. The visualization of the relative spatial relationship between the recommended entry point and the planned target is for example is at least one of a number, a text, a bar diagram, a progress bar or else.

For example, the navigation view comprises a visualization of a relative spatial relationship between the surgical instrument and at least one destination chosen from the recommended entry point and the planned target. For example, the navigation view comprises a visualization of a shortest distance between the trajectory and the planned target. The visualization of the relative spatial relationship between the surgical instrument and the at least one destination is for example at least one of a number, a text, a bar diagram, a progress bar or else.

The navigation view may comprise a visualization of a relationship between a distance between a position of the surgical instrument in the coordinate system and the recommended entry point and a distance between the recommended entry point and the target. The navigation view may comprise a visualization of a relationship between a first distance and a second distance, wherein the first distance is for example a distance between a position of the surgical instrument in the coordinate system and one of the recommended entry point and the target, and the second distance is for example a distance between the recommended entry point and the target. The navigation view in one example comprises a visualization of a fraction determined by dividing the first distance by the second distance. The navigation view may comprise a visualization of a fraction determined by dividing the second distance by the first distance. In another example, the navigation view comprises a visualization of at least one distance chosen from the first distance and the second distance.

The visualization of the relative spatial relationship, the visualization of the relationship and/or the visualization of the fraction is for example a progress bar. The progress bar may be concentrically formed around an alignment point in the navigation view. The alignment point in one example lies on a straight line between the recommended entry point and the planned target. The alignment point may be the center of the bullseye and/or the crosshairs.

In one variant, the recommended entry point is permanently changed from the planned entry point to the current entry point, upon the distance between the planned entry point and the current entry point fulfilling the predefined condition.

In another variant, the recommended entry point is instantaneously reset from the current entry point to the planned entry point, upon the distance between the planned entry point and the current entry point no longer fulfilling a predefined criterion. The predefined criterion is for example the predefined condition. In one example, the predefined criterion is that the distance between the planned entry point and the current entry point is lower than a limit value. The limit value defined by the predefined criterion may be larger than the threshold defined by the predefined condition. The limit value may be a predetermined value such as for example 0.5mm, 1mm, 1.5mm, 2mm, 2.5mm or 3 mm. The limit value in one example is larger than 0mm. The limit value may be obtained in a previous method step. The limit value may be set by a user. For example, the limit value is a value depending on a spatial relationship between the planned entry point and the target. In this case, the limit value may correspond to a certain percentage of the distance between the planned entry point and the target. The limit value is for example a value depending on a spatial relationship between the surgical instrument and at least one of the target, the current entry point and the planned entry point. The limit value may be chosen such that the instantaneous reset of the recommended entry point can be detected by a user solely from the navigation view. The limit value may be larger than a distance described by a single pixel of the navigation view. For example, the limit value is larger than a predetermined percentage of a width of a shaft of the surgical instrument.

According to a second aspect, a processor for a surgical navigation system is provided. The processor is configured to obtain planning data defining a planned entry point and a planned target, wherein the planned entry point and the planned target are defined in a coordinate system of a computer model of at least one anatomical element. The processor is further configured to obtain tracking data describing a current pose of a surgical instrument in the coordinate system and to determine, based on at least the tracking data, a current entry point lying on a trajectory having a fixed spatial relationship with the surgical instrument. The processor is configured to control display of a navigation view visualizing at least a recommended entry point, wherein the recommended entry point is instantaneously changed from the planned entry point to the current entry point, upon a distance between the planned entry point and the current entry point fulfilling a predefined condition.

According to a third aspect, a computer-readable medium is provided. The computer-readable medium comprises a computer program, the computer program comprising instructions which, when the program is executed by a processor, cause the processor to carry out the method of the first aspect.

According to a fourth aspect, a data carrier signal is provided. The data carrier signal carries information which represent a computer program, the computer program comprising instructions which, when the program is executed by a processor, cause the processor to carry out the method of the first aspect.

### Brief Description of the Drawings

Further details, advantages and aspects of the present disclosure will become apparent from the following embodiments taken in conjunction with the drawings wherein:
- Fig. 1: shows a surgical navigation system;
- Fig. 2: shows a method for controlling display of a navigation view;
- Fig. 3: shows a sagittal (lateral) view of a computer model of at least one anatomical element comprised in a patient's body with a planned entry point and a planned target;
- Fig. 4: shows a sagittal (lateral) view of the computer model and a trajectory of a surgical instrument in a first exemplary pose;
- Fig. 5: shows a sagittal (lateral) view of the computer model and the trajectory of the surgical instrument in a second exemplary pose;
- Fig. 6: shows a sagittal (lateral) view of the computer model and the trajectory of the surgical instrument in a third exemplary pose;
- Fig. 7: shows a sagittal (lateral) view of the computer model and the trajectory of the surgical instrument in a fourth exemplary pose;
- Fig. 8: shows a perspective view of the computer model with the surgical instrument in a fifth exemplary pose;
- Fig. 9: shows the navigation view with the surgical instrument in the fifth exemplary pose;
- Fig. 10: shows a sagittal (lateral) view of the computer model indicating a viewing direction of the navigation view and a trajectory of the surgical instrument in a sixth exemplary pose;
- Fig. 11: shows a first example of the navigation view with the surgical instrument in the sixth exemplary pose;
- Fig. 12: shows a sagittal (lateral) view of the computer model indicating the viewing direction of the navigation view and the trajectory of the surgical instrument in a seventh exemplary pose;
- Fig. 13: shows the first example of the navigation view with the surgical instrument in the seventh exemplary pose;
- Fig. 14: shows a sagittal (lateral) view of the computer model indicating the viewing direction of the navigation view and the trajectory of the surgical instrument in an eighth exemplary pose;
- Fig. 15: shows the first example of the navigation view with the surgical instrument in the eighth exemplary pose;
- Fig. 16: shows a sagittal (lateral) view of the computer model indicating the viewing direction of the navigation view and the trajectory of the surgical instrument in a ninth exemplary pose;
- Fig. 17: shows the first example of the navigation view with the surgical instrument in the ninth exemplary pose;
- Fig. 18: shows a second example of the navigation view with the surgical instrument in an orientation of the eighth exemplary pose;
- Fig. 19: shows the second example of the navigation view with the surgical instrument in the orientation of the eighth exemplary pose;
- Fig. 20: shows the second example of the navigation view with the surgical instrument in the orientation of the eighth exemplary pose;
- Fig. 21: shows the second example of the navigation view with the surgical instrument in the orientation of the eighth exemplary pose;
- Fig. 22: shows the second example of the navigation view with the surgical instrument in the orientation of the eighth exemplary pose;
- Fig. 23: shows different recommended entry points on the trajectory of the surgical instrument.

### Detailed Description

In the following description, exemplary embodiments of a computer-implemented method, a processor, a computer-readable medium and a data carried signal will be explained with reference to the drawings. The same reference numerals will be used to denote the same or similar structural features or method steps.

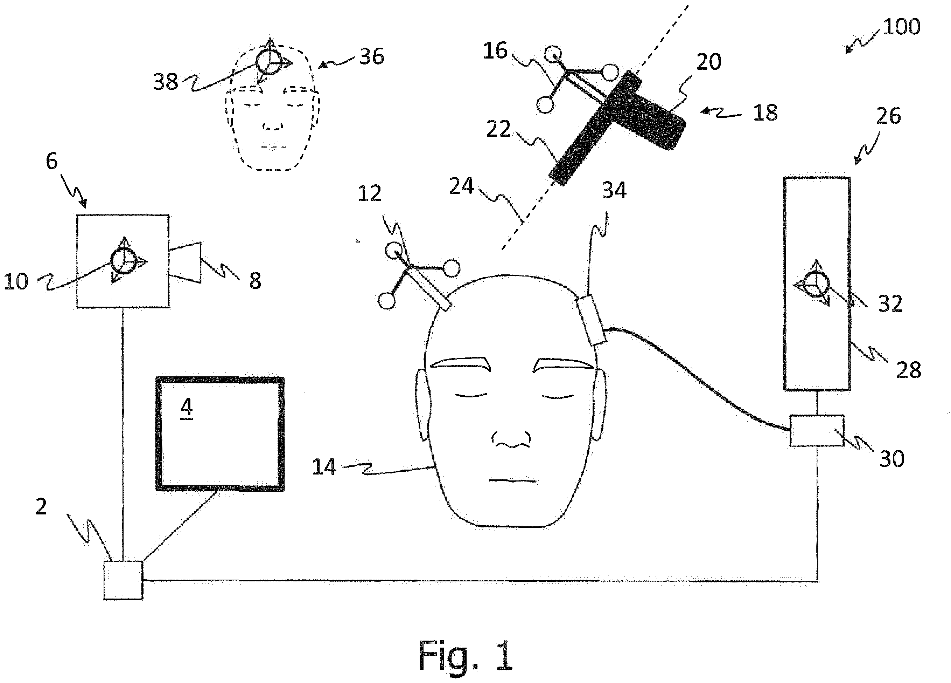

Fig. 1 shows a surgical navigation system 100. The surgical navigation system 100 comprises a processor 2 and a display 4. The surgical navigation system 100 further comprises an optical tracking system 6 including an optical sensor 8 and having a first tracking coordinate system 10. The optical tracking system 6 is configured to track optical trackers such as an optical tracker 12 attached to a patient 14 and an optical tracker 16 attached to a surgical instrument 18.

The surgical instrument 18 comprises a handle 20 and a shaft 22 having a longitudinal axis 24 that coincides with an insertion direction of the surgical instrument or with an insertion direction of a tool to be inserted through the shaft 22 of the surgical instrument 18.

The surgical navigation system 100 further comprises an electromagnetic tracking system 26 comprising a field generator 28 and a position determining unit 30. The position determining unit 30 is configured to determine a position of an electromagnetic sensor with respect to the field generator 28 in a second tracking coordinate system 32. For example, and electromagnetic sensor 34 is attached to the patient 14 and an additional electromagnetic sensor is attached to the surgical instrument 18 (not shown). The position determining unit 30 may be comprised in or realized by the processor 2. It is noted that the surgical navigation system 100 may comprise only one, or may comprise both of the optical tracking system 6 and the electromagnetic tracking system 26.

Patient image data 36 in an image data coordinate system 38 may be stored in a storage unit (not shown) and/or obtained by the processor 2. A computer model of at least one anatomical element may be determined based on the patient image data 36 by the processor 2, for example in the image data coordinate system 38. A registration can be performed in order to derive a transformation between a position and orientation of the optical tracker 12 and the image data coordinate system 38. Alternatively or additionally, a registration may be performed in order to derive a transformation between a position and orientation of the electromagnetic tracker 34 and the image data coordinate system 38. This enables the processor 2 to determine a correlation of the patient image data 36 with a current position and orientation of the patient 14. The surgical instrument 18 can be tracked as well in order to enable determining and displaying a navigation view indicating a relative spatial relationship between the surgical instrument 18 and the patient 14.

The navigation view may show a representation of the computer model, which was registered relative to the patient 14, and a representation of the surgical instrument 18. The determination of the navigation view and the controlling of the navigation view to be displayed on the display 4 may be performed by the processor 2. The navigation view is configured to help a surgeon during surgical navigation.

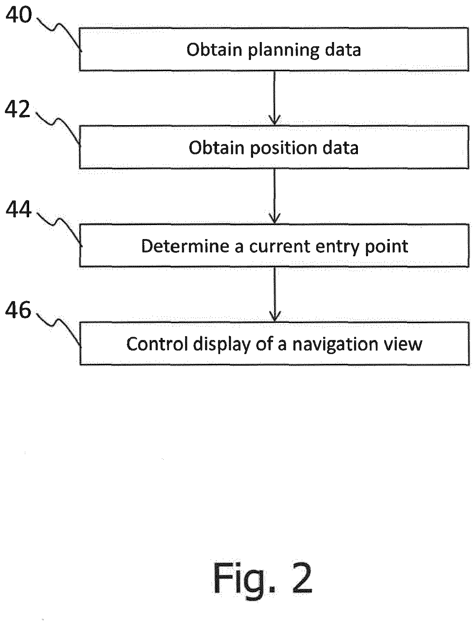

Fig. 2 shows a flow diagram of a method for controlling display of the navigation view. The method may be performed by the processor 2 of the surgical navigation system 100.

The method comprises a step 40 of obtaining planning data. The planning data describes a planned entry point in a coordinate system of the computer model of the at least one anatomical element. The planned entry point for example lies on an outer surface of a computer model of a patient body. The patient body is the body of the patient 14. The outer surface of the computer model of the at least one anatomical element may represent a bony surface of the at least one anatomical element. The planning data further describes a planned target in the coordinate system of the computer model. The planned target for example lies beneath the outer surface of the computer model. The planned target may be an anatomical element comprised in the patient body and identified in the patient image data 36. The planned target may comprise or be identical to a planned target point. The coordinate system of the computer model is in one example identical with the image data coordinate system 38.

The method comprises a further step 42 of obtaining tracking data. The tracking data describes a current pose of the surgical instrument 18 in the coordinate system. The current pose comprises at least one of a current position, also referred to as corresponding position, and a current orientation, also referred to as corresponding orientation, of the surgical instrument 18. The current orientation describes an alignment of the surgical instrument 18 in the coordinate system, for example three dimensional coordinates of the axis 24. The current position of the surgical instrument 18 may be described by a point on the axis 24 or by three-dimensional coordinates in the coordinate system.

In case the optical tracking system 6 determines the pose of the surgical instrument 18 by tracking the optical tracker 16 in the first tracking coordinate system 10, a known transformation between the first tracking coordinate system 10 and the coordinate system can be used in order to determine the current pose of the surgical instrument 18 in the coordinate system. This known transformation may be obtained by performing a registration as mentioned above.

In addition or alternatively to the optical tracking system 6 tracking the patient 14, the electromagnetic tracking system 26 may be used for this purpose. In case the electromagnetic tracking system 26 determines the pose of the surgical instrument 18 by tracking the electromagnetic tracker 34 in the second tracking coordinate system 32, a known transformation between the second tracking coordinate system 32 and the coordinate system can be used in order to determine the current pose of the surgical instrument 18 in the coordinate system. This known transformation may be obtained by performing a registration as mentioned above. In the following, all described (i.e., relative) positions and orientations are meant to be (i.e., relative) positions and orientations in the coordinate system. It is apparent that the (i.e., relative) positions and orientations may alternatively be described in a different coordinate system and that a transformation can be used to transform the coordinate system into the different coordinate system.

The method comprises a step 44 of determining, based on the tracking data, the current entry point. The current entry point is an approximation of where the surgical instrument will enter the at least one anatomical element, in case it is being inserted into the at least one anatomical element starting from the current pose. The current entry point lies on a trajectory having a fixed spatial relationship with the surgical instrument 18.

The current entry point in one example lies on a distal (front) tip of the surgical instrument. In this case, the trajectory does not necessarily need to be determined and the tracking data may only describe a position of the surgical instrument 18 in the current pose. In addition or alternatively thereto, the current entry point may lie on the outer surface of the computer model. In addition or alternatively thereto, the current entry point may be the point on the trajectory of the surgical instrument which is closest to the planned entry point.

In case the current entry point lies remote from the surgical instrument, the method may comprise determining trajectory data describing the trajectory of the surgical instrument 18. The trajectory is for example determined based on the tracking data describing at least an orientation of the surgical instrument 18 in the current pose. The trajectory is a straight line defined in the coordinate system and has a fixed spatial relationship with the surgical instrument 18. According to one example, the trajectory is parallel with or coincides with the longitudinal axis 24 of the surgical instrument 18. Further data may be taken into account for determining the trajectory data, for example geometry data describing the fixed spatial relationship.

The method also comprises a step 46 of controlling display of a navigation view. The navigation view visualizes at least a recommended entry point. For example, the navigation view visualizes the recommended entry point by being a view aligned or oriented with respect to the recommended entry point. A viewing direction of the navigation view may be determined based on the recommended entry point, so that the navigation view visualizes the recommended entry point. The navigation view may further visualize the planned target. In one example, the navigation view is a bullseye view centered on a straight line between the recommended entry point and the planned target and having a straight line between the recommended entry point and the target as a viewing direction. In a different example, the navigation view shows a perspective view of the three dimensional computer model of the at least one anatomical element comprised in the body of the patient 14, in which view the recommended entry point is highlighted.

The recommended entry point is instantaneously changed from the planned entry point to a current entry point lying on the trajectory, upon a distance between the planned entry point and the current entry point fulfilling a predefined condition. The predefined condition is for example that the distance between the planned entry point and the current entry point is lower than a threshold. The threshold may be a predetermined value above 0mm below 5mm, such as 2mm. For example, the threshold is a value depending on a spatial relationship between the planned target point and the target. In this case, the threshold may correspond to a certain percentage of the distance between the planned target point and the target.

In the following, details of the method of Fig. 2, which may be executed by the processor 2, are described with reference to Figs. 3 to 23.

Fig. 3 shows a sagittal (lateral) view of the computer model of a patient's skull with a planned entry point 48 and a planned target 50. A target point 52 is also shown. The target point 52 may be selected by a user during planning the target 50, or may be determined automatically after the target 50 has been planned (e.g., outlined by a surgeon on the display 4). In the latter case, the target point 52 is for example determined as a center point of the target 50 and/or as a point inside the target 50 being furthest from an outline or surface of the target 50. The target point 52 may lie on an outer surface of the planned target 50. A planned trajectory 54 extending between the planned entry point 48 and the planned target 50, in the shown example between the planned entry point 48 and the planned target point 52, can also be determined based on the planning data. The planned trajectory 54 may be determined automatically based on the planned entry point 48 and the planned target 50. The view shown in Fig. 3 may be determined solely based on the planning data, i.e., the planning data may comprise or be derived from the patient image data 36. In another example, the view shown in Fig. 3 is determined based on the patient image data 36 and the planning data.

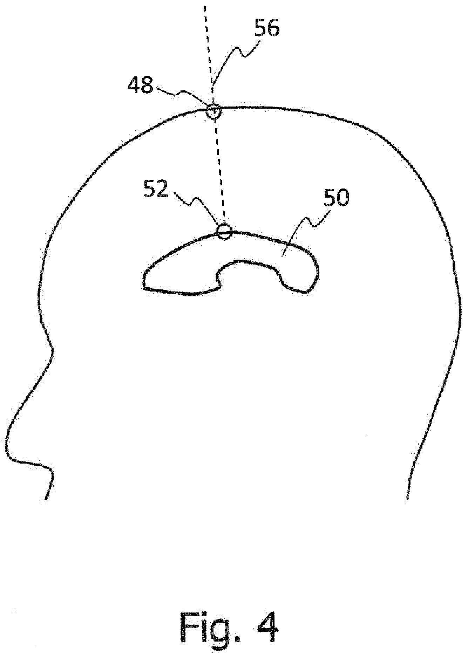

Fig. 4 shows a sagittal (lateral) view of the computer model and an actual, or current, trajectory 56 of the surgical instrument 18 in a first exemplary pose. The trajectory 56 can be determined based on the tracking data and has a fixed spatial relationship to the surgical instrument 18. The trajectory 56 is therefore indicative of the current pose of the surgical instrument 18, wherein the current pose in Fig. 4 is the first exemplary pose. The target point 52 in this example lies on the planned target 50. The shown view represents an ideal alignment of the surgical instrument 18 with respect to the patient 14. In particular, the trajectory 56 of the surgical instrument 18 runs through the planned entry point 48 and reaches the planned target point 52. In this case, the current pose of the surgical instrument 18 perfectly fits the planned target point 52 and the planned entry point 48. In other words, the trajectory 56 coincides with the planned trajectory 54.

Fig. 5 shows a sagittal (lateral) view of the computer model with the actual, or current, trajectory 56 of the surgical instrument 18 in a second exemplary pose. The second pose orientation results in the trajectory 56 extending through the planned entry point 48 but not through the planned target point 52. The trajectory 56 in this example still reaches the target 50, although that may not be the case if the target 50 is smaller or positioned differently. Such an alignment of the surgical instrument 18 with respect to the patient is disadvantageous, as the target point 52 will be missed by the surgical instrument 18 in case a surgeon follows the trajectory 56 with the surgical instrument 18.

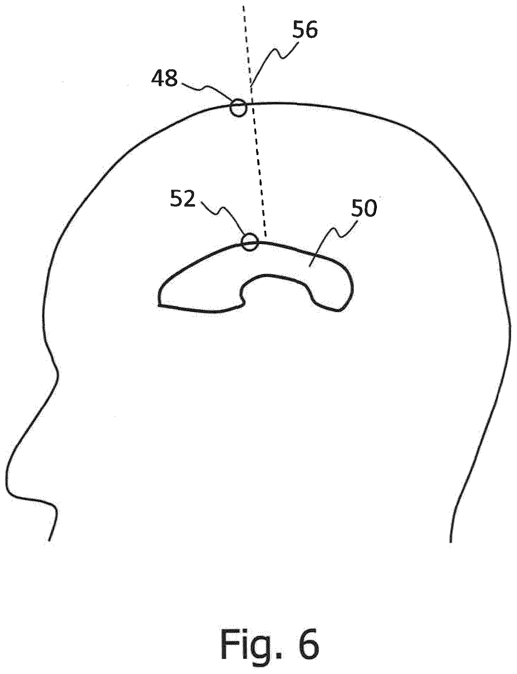

Fig. 6 shows a sagittal (lateral) view of the computer model with the trajectory 56 of the surgical instrument 18 in a third exemplary pose. The third exemplary pose results in the trajectory 56 extending essentially parallel to the planned trajectory 54, but not extending through the planned entry point 48 or the planned target point 56, although the trajectory 56 reaches the target 50. Such an alignment of the surgical instrument 18 with respect to the patient is disadvantageous as the target point 52 will be missed by the surgical instrument 18 in case a surgeon follows the trajectory 56 with the surgical instrument 18.

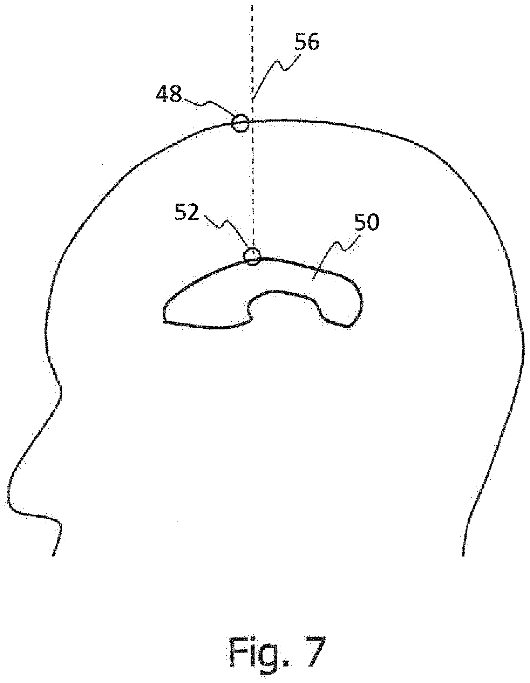

Fig. 7 shows a sagittal (lateral) view of the computer model with the trajectory 56 of the surgical instrument 18 in a fourth exemplary pose. The fourth exemplary pose results in the trajectory 56 extending to the planned target point 52, but not extending through the planned entry point 48. The target point 52 will be reached by the surgical instrument 18 in case a surgeon follows the trajectory 56 with the surgical instrument 18. As the planned target point 52 is much more important than the planned entry point 48, such an alignment of the surgical instrument 18 with respect to the patient will in most cases be acceptable. For example, the planned trajectory 54 may be planned such that critical anatomical structures are avoided by the planned trajectory 54. Therefore, the trajectory 56 should not deviate from the planned trajectory 54 too much. For that reason, the trajectory 56 shown in Fig. 7 is acceptable in case the distance between the planned entry point 48 and the current entry point lying on the trajectory 56 fulfills the predefined condition, for example falls below the predetermined threshold.

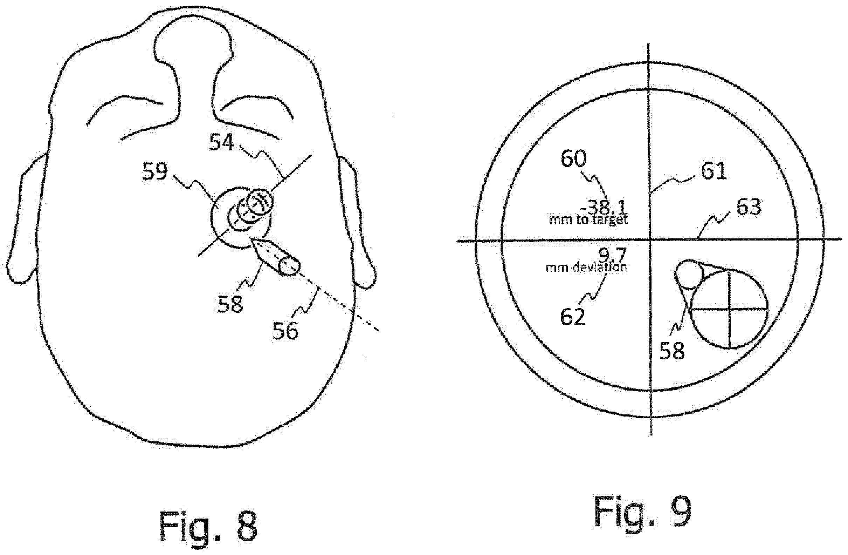

Fig. 8 shows a perspective view of the computer model in which the planned trajectory 54 and a representation 58 of a front tip, also referred to as distal tip, of the surgical instrument 18 is shown. The planned entry point 48 is visualized by an intersection between the planned trajectory 54 and a circular plane 59 tangential to the outer surface of the computer model. The surgical instrument 18 in this case is in a fifth exemplary pose. The orientation and position of the surgical instrument 18 in the fifth exemplary pose are known in the coordinate system. This enables a display of the representation 58 in relation to the computer model. As can be seen, the front tip of the surgical instrument 18 is remote from the planned trajectory 54 and thus also remote from the planned target point 48. In addition, there is a large angle between the trajectory 56 and the planned trajectory 54. This leads to a large distance between the trajectory 56 and the planned target point 52 (not shown).

Preferably, the perspective view shown in Fig. 8 is oriented such that it represents a current viewing direction of the surgeon onto the patient 14. The current viewing direction may be determined by tracking a position of the surgeon relative to the patient 14, using one or both of the tracking systems 6 and 26. Alternatively or additionally, the current viewing direction can be determined based on the fifth exemplary pose of the surgical instrument 18. In another variant, the current viewing direction is obtained from viewing direction information input by a user. In one variant, the perspective view shown in Fig. 8 is displayed together with or as the navigation view.

Fig. 9 shows an example of the navigation view with the surgical instrument 18 being in the fifth exemplary pose. The navigation view is in this case determined by the processor 2 based on the tracking data describing the fifth exemplary pose of the surgical instrument 18 comprising an orientation and position of the surgical instrument 18 in the coordinate system.

The navigation view in this example is a bullseye view having a straight line extending between the planned entry point 48 and the planned target point 52 as a viewing direction. The navigation view in this example comprises a pair of crosshairs 61 and 63. A first crosshair 61 extends essentially vertically whilst a second crosshair 63 extends essentially horizontally. Note that the bullseye view also comprises two circles formed concentrically around an alignment point. The alignment point coincides with an intersection of the two crosshairs 61 and 63. In other examples, the navigation view does not comprise such circles but comprises at least one of the crosshairs 61 and 63.

The straight line defining the viewing direction 64 of the navigation view extends between the planned entry point 48 and the planned target point 52. The bullseye view is centered on the straight line. The alignment point lies on the straight line.

Also, the representation 58 of the tip of the surgical instrument 18 is shown. The representation 58 in this case is a two-dimensional projection of the tip of the surgical instrument 18 into a plane perpendicular to viewing direction. Still further, an indication 60 of a distance between the distal tip of the surgical instrument 18 and the target 50 (i.e., the target point 52) and an indication 62 of a lowest distance between the trajectory 56 and the target 50 (i.e., the target point 52) are shown. An additional indication of a relative spatial relationship between the recommended entry point (in this example the planned entry point 48) and the target 50 may also be shown.

It is apparent from Fig. 9 that the trajectory 56 of the surgical instrument 18 in the fifth exemplary orientation is disadvantageous. A distance between the current entry point lying on the trajectory 56 of the surgical instrument 18 in the fifth exemplary orientation and the planned entry point 48 does not fulfill the predetermined condition. Therefore, in case the surgical instrument 18 is in the fifth exemplary pose, the navigation view controlled by the processor 2 to be displayed is a bullseye view centered on the straight line extending between the planned entry point 48 and the planned target point 52.

By continuously tracking the surgical instrument 18, the navigation view can be controlled by the controller 2 in near real-time. Controlling the navigation view preferably comprises determining the navigation view and triggering the display 4 to display the determined navigation view. As a result, the surgeon can be provided with an up-to date navigation view corresponding to the current pose of the surgical instrument.

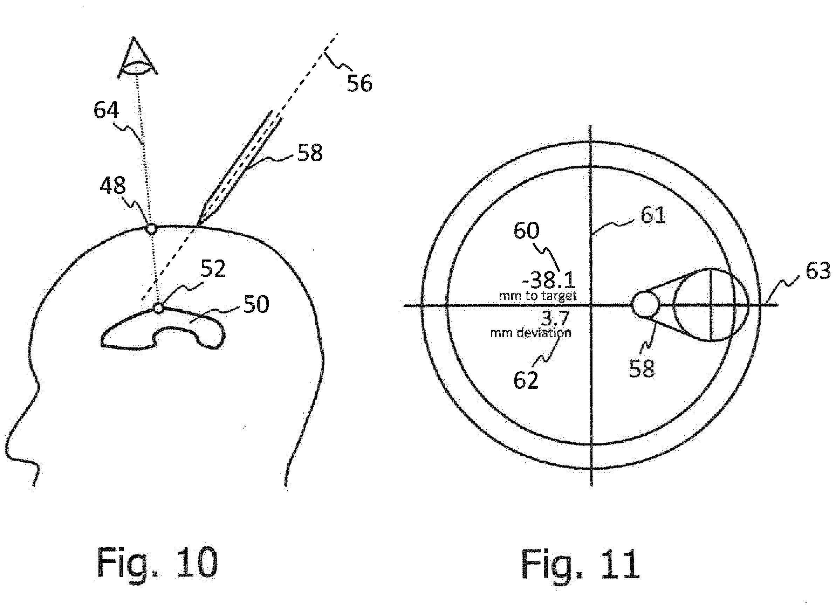

Figs. 10, 12, 14 and 16 show different relative positions between the surgical instrument 18, the planned entry point 48 and the planned target 50. These figures describe a sequential movement of the surgical instrument 18, namely a sequential movement from a sixth exemplary pose via a seventh exemplary pose and an eighth exemplary pose to a ninth exemplary pose. Figs. 11, 13, 15 and 17 show corresponding navigation views controlled by the processor 2 to be visualized on display 4 for the relative positional relationships shown in Figs. 10, 11, 12 and 14. In other words, Figs. 11, 13, 15 and 17 show a sequential change of the navigation view that corresponds to the sequential change in pose of the surgical instrument 18 from the sixth exemplary pose via the seventh exemplary pose and the eighth exemplary pose to the ninth exemplary pose. The representation 58 in Figs. 11, 13, 15 and 17 is a two-dimensional projection of the distal tip of the surgical instrument 18 into a plane perpendicular to viewing direction of the navigation view.

Fig. 10 shows a sagittal (lateral) view of the computer model indicating a viewing direction 64 of the navigation view. Also shown is the representation 58 of the distal tip of the surgical instrument 18 in the sixth exemplary pose. The representation 58 may be determined by the processor 2 based on the tracking data describing the sixth exemplary pose of the surgical instrument 18 in the coordinate system by describing a corresponding position and orientation of the surgical instrument 18 in the coordinate system. It can be seen that in this example, a straight line extending between the planned target point 48 and the planned target point 52 of the target 50 is used as the viewing direction of the navigation view. In this case, according the method described herein, display of the first example of the navigation view as shown in Fig. 11 may be controlled by the processor 2.

Fig. 11 shows a first example of the navigation view with the surgical instrument 18 being in the sixth exemplary pose. The navigation view is in this case determined by the processor 2 based on the tracking data describing the sixth exemplary pose of the surgical instrument 18 in the coordinate system by describing a corresponding position and orientation of the surgical instrument 18 in the coordinate system. As can be seen from the indication 62, the trajectory 56 of the surgical instrument 18 in the sixth exemplary pose is closer to the target point 52 than the trajectory 56 of the surgical instrument 18 in the fifth exemplary pose. A distance between the current entry point lying on the trajectory 56 of the surgical instrument 18 in the sixth exemplary pose and the planned entry point 48 does not fulfill the predetermined condition. Therefore, in case the surgical instrument 18 is in the sixth exemplary pose, the navigation view controlled by the processor 2 to be displayed is in this example a bullseye view centered on the straight line extending between the planned entry point 48 and the planned target point 52, as shown in Fig. 11.

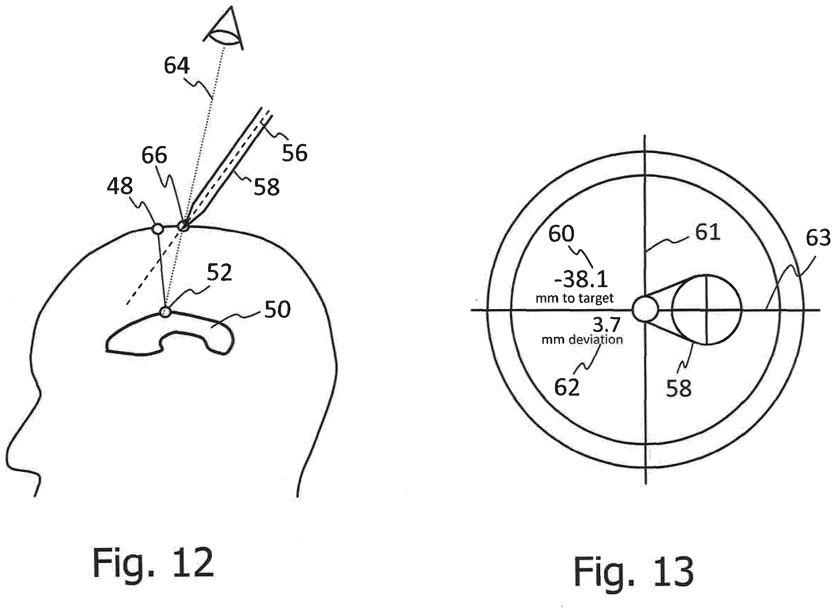

Fig. 12 shows a sagittal (lateral) view of the computer model indicating the viewing direction 64 of the navigation view. Also shown is the representation 58 of the distal tip of the surgical instrument 18 in the seventh exemplary pose. The representation 58 may be determined by the processor 2 based on the tracking data describing the seventh exemplary pose of the surgical instrument 18 in the coordinate system by describing a corresponding position and orientation of the surgical instrument 18 in the coordinate system. A distance between the current entry point 66 lying on the trajectory 56 of the surgical instrument 18 in the seventh exemplary pose and the planned entry point 48 does fulfill the predetermined condition. In the shown example, the current entry point 66 lies on the trajectory 56, on the distal tip of the surgical instrument 18, and on the outer surface of the computer model. It can be seen that in this example, the straight line extending between the current entry point 48 and the planned target point 52 of the target 50 is used as the viewing direction of the navigation view. In this case, according the method described herein, display of the example of the navigation view as shown in Fig. 13 may be controlled by the processor 2.

Fig. 13 shows an example of the navigation view with the surgical instrument 18 being in the seventh exemplary pose. The navigation view is in this case determined by the processor 2 based on the tracking data describing the seventh exemplary pose of the surgical instrument 18 in the coordinate system by describing a corresponding position and orientation of the surgical instrument 18 in the coordinate system. The distance between the current entry point 66 lying on the trajectory 56 of the surgical instrument 18 in the seventh exemplary pose and the planned entry point 48 does fulfill the predetermined condition. Therefore, in case the surgical instrument 18 is in the seventh exemplary pose, the navigation view controlled by the processor 2 to be displayed is in this example a bullseye view centered on the straight line extending between the current entry point 66 and the planned target point 52, as shown in Fig. 13. An additional indication of a relative spatial relationship between the recommended entry point (in this example the current entry point 66) and the target 50 may also be shown.

It is apparent from Figs. 10 to 13 that the navigation view controlled by the processor 2 to be displayed, on the display 4, is changed upon the distance between the current entry point and the planned entry point 48 fulfilling the predetermined condition. In particular, in the shown examples, the viewing direction 64 of the navigation view is instantaneously changed from a first viewing direction to a second viewing direction, wherein the first viewing direction coincides with the straight line extending between the planned entry point 48 and the planned target point 52, and the second viewing direction coincides with the straight line extending between the current entry point 66 and the planned target point 52. Therefore, once the surgical instrument is positioned sufficiently close to the planned entry point 48, the viewing direction 64 of the navigation view can be instantaneously changed, allowing an improved navigation of the surgical instrument 18. For instance, a surgeon need not perfectly hit the planned entry point 48 by positioning the surgical instrument 18 on the patient's body. Small deviations that are regarded as acceptable may therefore be defined as the threshold of the predefined condition.

When comparing the navigation view of Fig. 11 with the navigation view of Fig. 13, it can be seen that the representation 58 jumps from a position remote from the center of the bullseye to the center of the bullseye. This is because in this example of the navigation view, the viewing direction 64 is instantaneously changed upon the distance between the current entry point 66 and the planned entry point 48 fulfilling the predefined condition. The instantaneous (e.g., sudden, prompt, direct or single-step) change of the recommended entry point occurs when it is determined that the distance between the current entry point 66 and the planned entry point 48 fulfills the predefined condition. By continuously tracking a pose of the surgical instrument 18 and the patient 14 and determining their respective orientations and/or positions in the coordinate system, the aforementioned distance can be continuously calculated. Once the calculated distance fulfills the predefined condition, the recommended entry point is instantaneously changed.

In case a perspective view such as the one described above with reference to Fig. 8 is used as or in addition to the navigation view, the visualized recommended entry point can also be instantaneously changed from the planned entry point 48 to the current entry point 66 upon the distance between the current entry point 66 and the planned entry point 48 fulfilling the predefined condition. The current entry point 66 may then be visualized by visually highlighting it in the perspective view or by showing the trajectory 56 intersecting a plane tangential to the outer surface of the computer model and orthogonal to the trajectory 56.

After instantaneously changing the recommended entry point, it may be reset from the current entry point 66 to the planned entry point 48 upon the distance between the planned entry point 48 and the current entry point 48 no longer fulfilling a predefined criterion such as the predefined condition. Alternatively, the recommended entry point may be permanently changed from the planned entry point 48 to the current entry point 66, upon the distance between the planned entry point 48 and the current entry point 66 fulfilling the predefined condition.

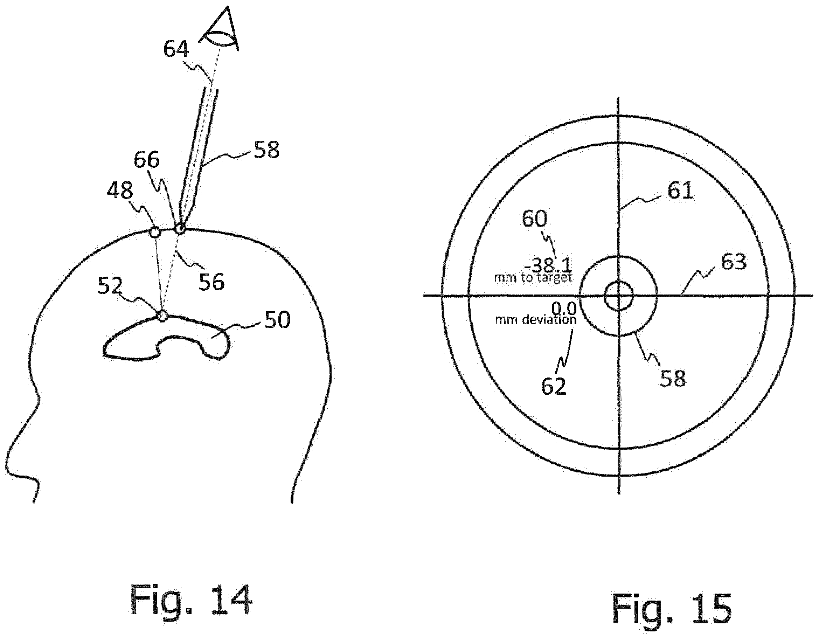

Fig. 14 shows a sagittal (lateral) view of the computer model indicating the viewing direction 64 of the navigation view. Also shown is the representation 58 of the tip of the surgical instrument 18 in an eighth exemplary pose. The representation 58 may be determined by the processor 2 based on the tracking data describing the eighth exemplary pose of the surgical instrument 18 in the coordinate system by describing the corresponding position and orientation of the surgical instrument 18 in the coordinate system. The current entry point 66 of Fig. 14 is the same as the current entry point 66 shown in Fig. 12. Therefore, the distance between the current entry point 66 lying on the trajectory 56 of the surgical instrument 18 in the eighth exemplary pose and the planned entry point 48 does fulfill the predetermined condition, and, in case the surgical instrument 18 is in the eighth exemplary pose, the navigation view controlled by the processor 2 to be displayed may be a bullseye view centered on the straight line extending between the current entry point 66 and the planned target point 52. It can be seen that the trajectory 56 is aligned with the viewing direction 64 and coincides with the straight line between the current entry point 66 and the target point 52.

In order to guide a surgeon to move the surgical instrument 18 so as to align the trajectory 56 with the straight line extending between the current entry point 66 and the planned target point 52, the navigation view shown in Fig. 13 may be controlled to be displayed. After the alignment, the navigation view of Fig. 15 may be controlled to be displayed.

Fig. 15 shows an example of the navigation view with the surgical instrument 18 being in the eighth exemplary pose. As can be seen, the representation 58 is centered on the center of the bullseye view. In other words, the viewing direction 64 of the bullseye view corresponds to the orientation direction of the longitudinal axis 24 of the shaft 22 of the surgical instrument 18, the tip of which is shown as the representation 58. As the trajectory 56 in this case hits the planned target point 52, the indication 62 describes a deviation of 0 mm. The indication 60 in this case still shows a value of -38.1mm as the distance between the tip of the surgical instrument 18 and the target point 52.

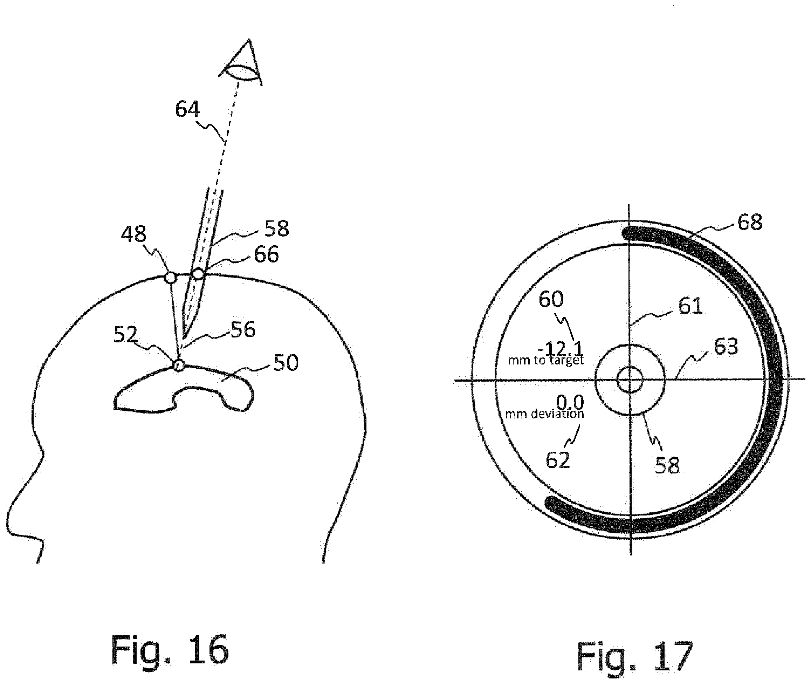

Fig. 16 shows a sagittal (lateral) view of the computer model indicating the viewing direction 64 of the navigation view. Also shown is the representation 58 of the tip of the surgical instrument 18 in the ninth exemplary pose. The representation 58 may be determined by the processor 2 based on the tracking data describing the ninth exemplary pose of the surgical instrument 18 in the coordinate system by describing a corresponding position and orientation of the surgical instrument 18 in the coordinate system. It can be seen that, although the orientation of the surgical instrument 18 is the same in Figs. 14 and 16, the position of the tip of the surgical instrument 18 differs between these two figures. In Fig. 14, the tip of the surgical instrument 18 lies on the outer surface of the computer model. In Fig. 16, the tip of the surgical instrument 18 lies underneath the outer surface of the computer model.

The current entry point 66 shown in Fig. 16 is the same as shown in Figs. 12 and 14. Therefore, the distance between the current entry point 66 lying on the trajectory 56 of the surgical instrument 18 in the ninth exemplary pose and the planned entry point 48 does fulfill the predetermined condition, and, in case the surgical instrument 18 is in the ninth exemplary pose, the navigation view controlled by the processor 2 to be displayed is a bullseye view centered on the straight line extending between the current entry point 66 and the planned target point 52. It can be seen that the trajectory 56 is aligned with the viewing direction 64 and extends between the current entry point 56 and the target point 52.

Fig. 17 shows an example of the navigation view with the surgical instrument 18 being in the ninth exemplary pose. As can be seen, the representation 58 is centered on the center of the bullseye view. In other words, the viewing direction 64 of the bullseye view corresponds to the direction of the longitudinal axis 24 of the shaft 22 of the surgical instrument 18, the tip of which is shown as the representation 58. As the trajectory 56 in this case hits the planned target point 52, the indication 62 indicates a deviation of 0 mm. In order to guide a surgeon to move the surgical instrument 18 so as to reach the target 50, the indication 60 is shown. The indication 60 in this case shows a value of -12.1mm as the distance between the tip of the surgical instrument 18 and the target point 52. This is because, compared to Figs. 14 and 15, the position of the tip of the surgical instrument 18 is closer to the target 50 in Figs. 16 and 17.

Furthermore, the navigation view in this example comprises a progress bar 68 concentrically formed around the straight line between the current entry point 66 and the target point 52. The progress bar indicates a distance between the tip of the surgical instrument 18 and the planned target 50 (i.e., the planned target point 52). In particular, the progress bar 68 is an indication of a relationship between the distance between the tip of the surgical instrument 18 and the current entry point 66, and the distance between the current entry point 66 and the target point 52. The closer the tip of the surgical instrument 18 to the target 50 is, the fuller (i.e., the more complete) is the progress bar. In another variant, the progress bar may be referred to as a countdown bar. In this case, the closer the tip of the surgical instrument 18 to the target 50 is, the emptier (i.e., the less complete) is the progress bar.

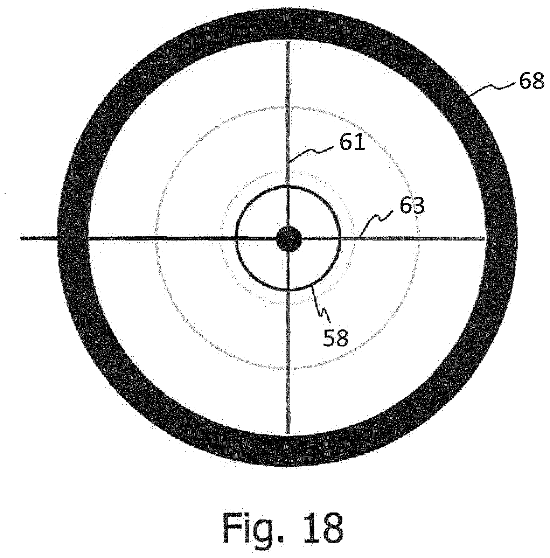

Figs. 18 to 22 show a second example of the navigation view having the straight line between the current entry point 66 and the target point 52 as the viewing direction. These Figures show the progress bar 68 in different states. In the second example of the navigation view, the progress bar 68 is the countdown bar.

Fig. 18 shows the navigation view with the progress bar 68 in case the tip of the surgical instrument 18 lies on the current entry point 66. In this case, the surgical instrument 18 is in the eighth exemplary pose.

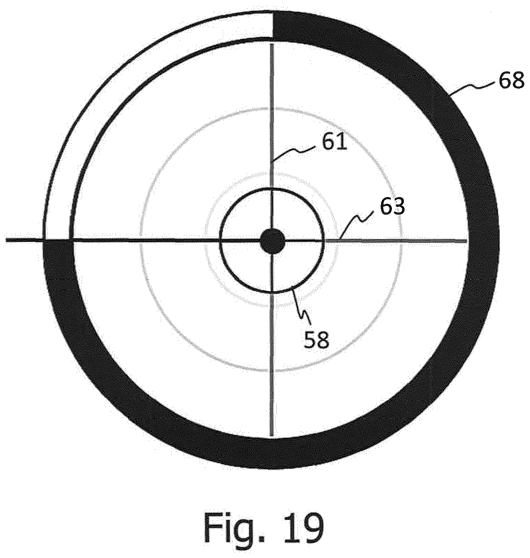

Fig. 19 shows the navigation view with the progress bar 68 indicating a completeness of insertion of the surgical instrument of 25%. In other words, the progress bar 68 in Fig. 19 indicates that the distance between the tip of the surgical instrument 18 and the current entry point 66 is 25% of the distance between the current entry point 66 and the target point 52. In this case, the surgical instrument 18 is the same orientation as in the eighth exemplary pose but in a different position as in the eighth exemplary pose. In other words, the trajectory 56 of the surgical instrument 18 is the same in Figs. 18 and 19.

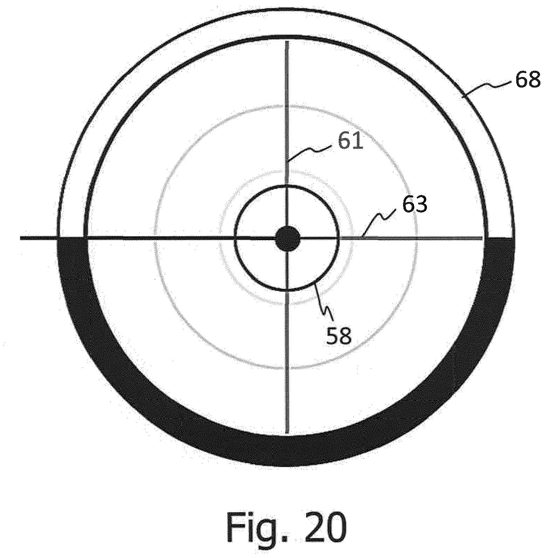

Fig. 20 shows the navigation view with the progress bar 68 indicating a completeness of insertion of the surgical instrument of 50%. In other words, the progress bar 68 in Fig. 20 indicates that the distance between the tip of the surgical instrument 18 and the current entry point 66 is 50% of the distance between the current entry point 66 and the target point 52. In this case, the surgical instrument 18 is in the same orientation as in the eighth exemplary pose but in a different position than in Fig. 18 or 19.

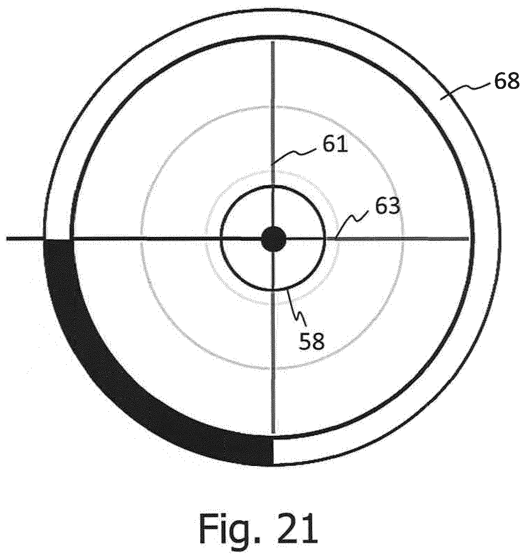

Fig. 21 shows the navigation view with the progress bar 68 indicating a completeness of insertion of the surgical instrument of 75%. In other words, the progress bar 68 in Fig. 21 indicates that the distance between the tip of the surgical instrument 18 and the current entry point 66 is 75% of the distance between the current entry point 66 and the target point 52. In this case, the surgical instrument 18 is in the same orientation as in the eighth exemplary pose but in a different position than in Figs. 18 to 20.

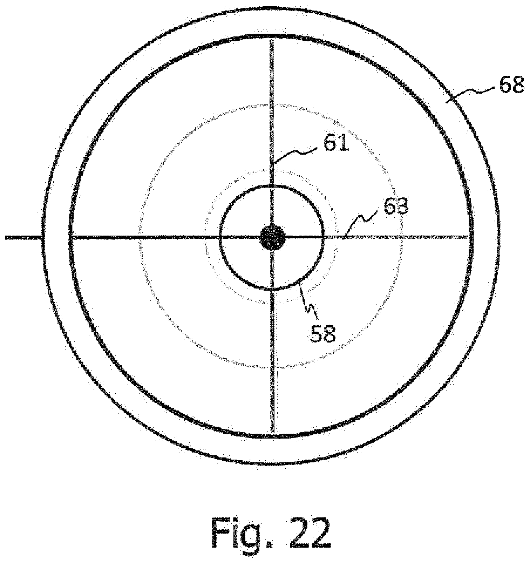

Fig. 22 shows the navigation view with the progress bar 68 indicating a completeness of insertion of the surgical instrument of 100%. In other words, the progress bar 68 in Fig. 22 indicates that the distance between the tip of the surgical instrument 18 and the current entry point 66 is 100% of the distance between the current entry point 66 and the target point 52. In this case, the surgical instrument 18 is in the same orientation as in the eighth exemplary pose but in a different position than in Figs. 18 to 21.

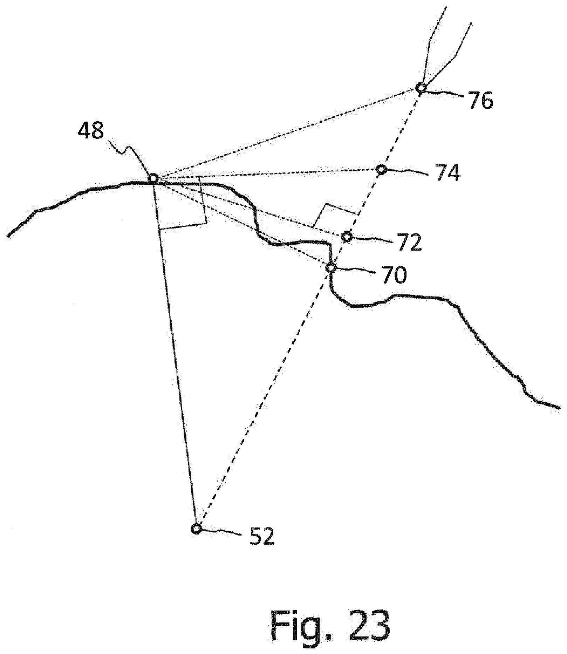

Fig. 23 shows different current entry points which by definition all lay on the trajectory 56 of the surgical instrument 18. A first point 70 lies on the outer surface of the computer model. A second point 72 is a point closest to the planned entry point 48. A third point 74 is a point lying in a plane extending through the planned entry point 48, the plane having the straight line between the planned entry point 48 and the target point 52 as a normal. A fourth point 76 lies on the tip of the surgical instrument 18. The current entry point 66 fulfills at least one requirement chosen from: the current entry point 66 lies on a (i.e. front) tip of the surgical instrument 18; the current entry 66 point lies on the outer surface of the computer model; the current entry point 66 is closest to the planned entry point 48 among any point lying on the trajectory 56; and the current entry 66 point lies in a plane, wherein the plane has a normal defined by a straight line between the planned entry point 48 and the planned target 50 (i.e., the planned target point 52), and wherein the planned entry point 48 lies in the plane. In the present embodiment, the current entry point is the second point 72, in particular, the point on the trajectory which lies closest to the planned entry point 48.

Also provided is a computer-readable medium comprising a computer program comprising instructions which, when executed by a processor (i.e., the processor 2), causes the processor to carry out the method described herein. Furthermore, a data carrier signal carrying information which represent the computer program is provided.

As described with reference to the exemplary embodiments, the technique presented herein in some variants enables displaying visual cues to decompose a surgeon's task of aligning the surgical instrument 18 into two sequential tasks. In a first task, the front tip of the surgical instrument 18 is aligned with the planned entry point 48. Afterwards, in a second task, the surgical instrument 18 is aligned such that the trajectory 56 passes through the planned target 50.

The navigation view is for example a bullseye view looking along the actual instrument trajectory 56. The bullseye view can be displayed together with the perspective view described above, which is a two or three dimensional visualization of the computer model.

The perspective view may be oriented such that it best matches an actual perspective of a surgeon onto the patient 14 lying in front of him. The orientation of the perspective view may be determined based on information from the optical tracking system 6 or the electromagnetic tracking system 26, or from both. The orientation of the perspective view may be further determined based on at least one known coordinate transformation and/or a known surgeon position relative to an operating table on which the patient 14 is positioned. This visualization by the perspective view enables the surgeon to easily find the planned entry point 48 on the patient 14.

The bullseye view showing the representation 58 of the front tip of the surgical instrument 18 may also show an arbitrary proximal point of the surgical instrument's longitudinal axis 24. As shown in the examples of Figs. 10 and 11, initially the bullseye view looks exactly along the planned trajectory 54. For example, when the front tip of the surgical instrument 18 is positioned less than an application specific distance from the planned trajectory (e.g. 2 mm), the bullseye view changes such that it is now looking along a straight line going through the current entry point 66 towards the target 50 as shown in Figs. 12 and 13. The center of the bullseye view in this example thus jumps from the planned entry point 48 to the current entry point 66.

The surgeon can then align the orientation of the surgical instrument 18 using the newly oriented bullseye view until the trajectory 56 passes through the target 50 as shown in Figs. 14 and 15, and then move the surgical instrument 18 towards the target 50 as shown in Figs. 16 and 17.

In most cases, it is more important to exactly hit the target point 52, whereas an offset of a few millimeters from the entry point 48 is usually inconsequential. This means that an alignment as shown in Fig. 7 is advantageous over the alignments shown in Figs. 5 and 6, where the trajectory 56 may slightly miss the planned entry point 48, but the trajectory 56 passes very close to the planned target point 52. Therefore, the method described herein supports the surgeon to first achieve a "good enough" alignment of the surgical instrument 18 to the planned entry point 48, and then an accurate alignment of the surgical instrument 18 so that the trajectory 56 accurately hits the target point 52.

This advantageous sequential alignment can be enabled by switching the navigation view from looking from the planned entry point 48 towards the target 50 to look from the current entry point 66 towards the target 50, once the current entry point 66 is "good enough", so that the surgeon can then focus fully on correcting the alignment of the surgical instrument 18 with respect to the patient 14 so that the trajectory 56 hits the target 50.

The method described herein, which instantaneously changes the recommended entry point, has an advantage compared with simply orienting or aligning a bullseye view at the front tip of the surgical instrument 18 with a viewing direction looking towards the target 50, as it avoids indicating very large directional offsets when the tip comes near the target 50. Instead, the current entry point 66 can be considered as a pivot point about which the surgeon can make corrections to the orientation of the surgical instrument 18 and thus to the direction of the trajectory 56.

A combination of the perspective view, which is a surgeon-oriented two or three dimensional view, and the bullseye view may be displayed. Also, the bullseye view may be switched from looking from the planned entry point 48 towards the target 50 (Figs. 10, 11) to look from the current entry point 66 towards the target 50 (Figs. 12 and 13), once the current entry point 66 is "good enough", so that the surgeon can then focus fully on correcting the orientation of the surgical instrument 18 by pivoting the surgical instrument 18 on the current entry point 66 such that the trajectory 56 hits the target 50.

By instantaneously changing the recommended entry point from the planned entry point 48 to the current entry point 66, surgical navigation is improved. For example, a surgeon may not be able to or may decide to not position the surgical instrument 18 precisely on the planned entry point 48. In this case, a navigation view guiding the surgeon from the current entry point 66 to the target 50 may be advantageous. By only changing the recommended entry point to the current entry point 66 upon a distance between the current entry point 66 and the planned entry point 48 fulfilling a predetermined condition, it can be ensured that the trajectory 56 of the surgical instrument 18 is still sufficiently close to the planned trajectory 54.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A computer-implemented method of controlling display of a navigation view for surgical navigation, the method comprising:
obtaining (40) planning data defining a planned entry point (48) and a planned target (50), wherein the planned entry point (48) and the planned target (50) are defined in a coordinate system of a computer model of at least one anatomical element;
obtaining (42) tracking data describing a current pose of a surgical instrument (18) in the coordinate system;
determining, based on at least the tracking data, a current entry point (66) lying on a trajectory (56) having a fixed spatial relationship with the surgical instrument (18); and
**characterised in that** the method comprises controlling (46) display of a navigation view visualizing at least a recommended entry point, wherein the recommended entry point is instantaneously changed from the planned entry point (48) to the current entry point (66), upon a distance between the planned entry point (48) and the current entry point (66) fulfilling a predefined condition.

2. The method of claim 1, wherein the predefined condition is that the distance between the planned entry point (48) and the current entry point (66) is lower than a threshold.

3. The method of claim 1 or 2, wherein the navigation view visualizes the recommended entry point and the planned target (50).

4. The method of any of the preceding claims, wherein the navigation view has a viewing direction corresponding to a straight line between the recommended entry point and the planned target (50).

5. The method of any of the preceding claims, wherein the navigation view is a bullseye view centered on a straight line between the recommended entry point and the planned target (50).

6. The method of any of the preceding claims, wherein the navigation view comprises a visualization of a relative spatial relationship between the surgical instrument (18) and at least one destination chosen from the recommended entry point and the planned target (50).

7. The method of any of the preceding claims, wherein the navigation view comprises a visualization of a fraction determined by dividing a first distance by a second distance or the second distance by the first distance,
wherein the first distance is a distance between a position of the surgical instrument (18) in the coordinate system and one of the recommended entry point and the target (50), and
wherein the second distance is a distance between the recommended entry point and the target (50).

8. The method of claim 7, wherein the visualization of the fraction is a progress bar.

9. The method of claim 8, wherein the progress bar is concentrically formed around an alignment point in the navigation view.

10. The method of claim 9, wherein the alignment point lies on a straight line between the recommended entry point and the planned target (50).

11. The method of any of the preceding claims, wherein the recommended entry point is permanently changed from the planned entry point (48) to the current entry point (66), upon the distance between the planned entry point (48) and the current entry point (66) fulfilling the predefined condition.

12. The method of any of claim 1 to 10, wherein the recommended entry point is instantaneously reset from the current entry point (66) to the planned entry point (48), upon the distance between the planned entry point (48) and the current entry point (66) no longer fulfilling a predefined criterion.

13. A processor for a surgical navigation system, the processor configured to:
obtain (40) planning data defining a planned entry point (48) and a planned target (50), wherein the planned entry point (48) and the planned target (50) are defined in a coordinate system of a computer model of at least one anatomical element;
obtain (42) tracking data describing a current pose of a surgical instrument (18) in the coordinate system;
determine (44), based on at least the tracking data, a current entry point (66) lying on a trajectory (56) having a fixed spatial relationship with the surgical instrument (18); and
**characterised in that** the processor is configured to control (46) display of a navigation view visualizing at least a recommended entry point, wherein the recommended entry point is instantaneously changed from the planned entry point (48) to the current entry point (66), upon a distance between the planned entry point (48) and the current entry point (66) fulfilling a predefined condition.

14. A computer-readable medium comprising a computer program, the computer program comprising instructions which, when the program is executed by a processor, cause the processor to carry out the method of one of claims 1 to 12.

15. A data carrier signal carrying information which represent a computer program, the computer program comprising instructions which, when the program is executed by a processor, cause the processor to carry out the method of one of claims 1 to 12.

## Patentansprüche

1. Computer-implementiertes Verfahren zur Steuerung der Anzeige einer Navigationsansicht für die chirurgische Navigation, das Verfahren umfassend:
Erhalten (40) von Planungsdaten, die einen geplanten Eintrittspunkt (48) und ein geplantes Ziel (50) definieren, wobei der geplante Eintrittspunkt (48) und das geplante Ziel (50) in einem Koordinatensystem eines Computermodells von mindestens einem anatomischen Element definiert sind;
Erhalten (42) von Tracking-Daten, die eine aktuelle Pose eines chirurgischen Instruments (18) in dem Koordinatensystem beschreiben;
Bestimmen, basierend zumindest auf den Tracking-Daten, eines aktuellen Eintrittspunkts (66), der auf einer Trajektorie (56) liegt, die eine feste räumliche Beziehung zu dem chirurgischen Instrument (18) aufweist; und
**dadurch gekennzeichnet, dass** das Verfahren ein Steuern (46) der Anzeige einer Navigationsansicht umfasst, die zumindest einen empfohlenen Eintrittspunkt visualisiert, wobei der empfohlene Eintrittspunkt unverzüglich von dem geplanten Eintrittspunkt (48) zu dem aktuellen Eintrittspunkt (66) geändert wird, wenn ein Abstand zwischen dem geplanten Eintrittspunkt (48) und dem aktuellen Eintrittspunkt (66) eine vordefinierte Bedingung erfüllt.

2. Verfahren nach Anspruch 1, wobei die vordefinierte Bedingung darin besteht, dass der Abstand zwischen dem geplanten Eintrittspunkt (48) und dem aktuellen Eintrittspunkt (66) kleiner als ein Schwellenwert ist.

3. Verfahren nach Anspruch 1 oder 2, wobei die Navigationsansicht den empfohlenen Eintrittspunkt und das geplante Ziel (50) visualisiert.

4. Verfahren nach einem der vorhergehenden Ansprüche, wobei die Navigationsansicht eine Blickrichtung aufweist, die einer geraden Linie zwischen dem empfohlenen Eintrittspunkt und dem geplanten Ziel (50) entspricht.

5. Verfahren nach einem der vorhergehenden Ansprüche, wobei die Navigationsansicht eine Zielscheibenansicht ist, die auf einer geraden Linie zwischen dem empfohlenen Eintrittspunkt und dem geplanten Ziel (50) zentriert ist.

6. Verfahren nach einem der vorhergehenden Ansprüche, wobei die Navigationsansicht eine Visualisierung einer relativen räumlichen Beziehung zwischen dem chirurgischen Instrument (18) und mindestens einem Zielort umfasst, der aus dem empfohlenen Eintrittspunkt und dem geplanten Ziel (50) ausgewählt ist.

7. Verfahren nach einem der vorhergehenden Ansprüche, wobei die Navigationsansicht eine Visualisierung eines Bruchteils umfasst, der durch Division eines ersten Abstands durch einen zweiten Abstand oder des zweiten Abstands durch den ersten Abstand bestimmt wird,
wobei der erste Abstand ein Abstand zwischen einer Position des chirurgischen Instruments (18) in dem Koordinatensystem und einem von dem empfohlenen Eintrittspunkt und dem Ziel (50) ist, und
wobei der zweite Abstand ein Abstand zwischen dem empfohlenen Eintrittspunkt und dem Ziel (50) ist.

8. Verfahren nach Anspruch 7, wobei die Visualisierung des Bruchteils ein Fortschrittsbalken ist.

9. Verfahren nach Anspruch 8, wobei der Fortschrittsbalken konzentrisch um einen Ausrichtungspunkt in der Navigationsansicht geformt ist.

10. Verfahren nach Anspruch 9, wobei der Ausrichtungspunkt auf einer geraden Linie zwischen dem empfohlenen Eintrittspunkt und dem geplanten Ziel (50) liegt.

11. Verfahren nach einem der vorhergehenden Ansprüche, wobei der empfohlene Eintrittspunkt permanent von dem geplanten Eintrittspunkt (48) zu dem aktuellen Eintrittspunkt (66) geändert wird, wenn der Abstand zwischen dem geplanten Eintrittspunkt (48) und dem aktuellen Eintrittspunkt (66) die vordefinierte Bedingung erfüllt.

12. Verfahren nach einem der Ansprüche 1 bis 10, wobei der empfohlene Eintrittspunkt unverzüglich von dem aktuellen Eintrittspunkt (66) auf den geplanten Eintrittspunkt (48) zurückgesetzt wird, wenn der Abstand zwischen dem geplanten Eintrittspunkt (48) und dem aktuellen Eintrittspunkt (66) ein vordefiniertes Kriterium nicht mehr erfüllt.

13. Prozessor für ein chirurgisches Navigationssystem, wobei der Prozessor dazu eingerichtet ist:
Planungsdaten zu erhalten (40), die einen geplanten Eintrittspunkt (48) und ein geplantes Ziel (50) definieren, wobei der geplante Eintrittspunkt (48) und das geplante Ziel (50) in einem Koordinatensystem eines Computermodells von mindestens einem anatomischen Element definiert sind;
Tracking-Daten zu erhalten (42), die eine aktuelle Pose eines chirurgischen Instruments (18) in dem Koordinatensystem beschreiben;
basierend zumindest auf den Tracking-Daten einen aktuellen Eintrittspunkt (66) zu bestimmen (44), der auf einer Trajektorie (56) liegt, die eine feste räumliche Beziehung zu dem chirurgischen Instrument (18) aufweist; und
**dadurch gekennzeichnet, dass** der Prozessor dazu eingerichtet ist, eine Anzeige einer Navigationsansicht zu steuern (46), die zumindest einen empfohlenen Eintrittspunkt visualisiert, wobei der empfohlene Eintrittspunkt unmittelbar von dem geplanten Eintrittspunkt (48) zu dem aktuellen Eintrittspunkt (66) geändert wird, wenn ein Abstand zwischen dem geplanten Eintrittspunkt (48) und dem aktuellen Eintrittspunkt (66) eine vordefinierte Bedingung erfüllt.

14. Computerlesbares Medium, welches ein Computerprogramm umfasst, wobei das Computerprogramm Anweisungen enthält, die, wenn das Programm von einem Prozessor ausgeführt wird, den Prozessor veranlassen, das Verfahren nach einem der Ansprüche 1 bis 12 auszuführen.

15. Datenträgersignal, das Informationen trägt, die ein Computerprogramm repräsentieren, wobei das Computerprogramm Anweisungen enthält, die, wenn das Programm von einem Prozessor ausgeführt wird, den Prozessor veranlassen, das Verfahren nach einem der Ansprüche 1 bis 12 auszuführen.

## Revendications

1. Procédé mis en œuvre par ordinateur de commande d'affichage d'une vue de navigation pour une navigation chirurgicale, le procédé comprenant :
l'obtention (40) de données de planification définissant un point d'entrée planifié (48) et une cible planifiée (50), dans lequel le point d'entrée planifié (48) et la cible planifiée (50) sont définis dans un système de coordonnées d'un modèle informatique d'au moins un élément anatomique ;
l'obtention (42) de données de suivi décrivant une pose actuelle d'un instrument chirurgical (18) dans le système de coordonnées ;
la détermination, sur la base d'au moins les données de suivi, d'un point d'entrée actuel (66) se trouvant sur une trajectoire (56) ayant une relation spatiale fixe avec l'instrument chirurgical (18) ; et
**caractérisé en ce que** le procédé comprend la commande (46) d'un affichage d'une vue de navigation visualisant au moins un point d'entrée recommandé, dans lequel le point d'entrée recommandé est instantanément passé du point d'entrée planifié (48) au point d'entrée actuel (66), lorsqu'une distance entre le point d'entrée planifié (48) et le point d'entrée actuel (66) satisfait à une condition prédéfinie.

2. Procédé selon la revendication 1, dans lequel la condition prédéfinie consiste dans le fait que la distance entre le point d'entrée planifié (48) et le point d'entrée actuel (66) est inférieure à un seuil.

3. Procédé selon la revendication 1 ou 2, dans lequel la vue de navigation visualise le point d'entrée recommandé et la cible planifiée (50).

4. Procédé selon l'une quelconque des revendications précédentes, dans lequel la vue de navigation a une direction de visualisation correspondant à une ligne droite entre le point d'entrée recommandé et la cible planifiée (50).

5. Procédé selon l'une quelconque des revendications précédentes, dans lequel la vue de navigation est une vue en ultra-grand angulaire centrée sur une ligne droite entre le point d'entrée recommandé et la cible planifiée (50).

6. Procédé selon l'une quelconque des revendications précédentes, dans lequel la vue de navigation comprend une visualisation d'une relation spatiale relative entre l'instrument chirurgical (18) et au moins une destination choisie parmi le point d'entrée recommandé et la cible planifiée (50).

7. Procédé selon l'une quelconque des revendications précédentes, dans lequel la vue de navigation comprend une visualisation d'une fraction déterminée en divisant une première distance par une deuxième distance ou la deuxième distance par la première distance,
dans lequel la première distance est une distance entre une position de l'instrument chirurgical (18) dans le système de coordonnées et un(e) parmi le point d'entrée recommandé et la cible planifiée (50), et
dans lequel la deuxième distance est une distance entre le point d'entrée recommandé et la cible planifiée (50).

8. Procédé selon la revendication 7, dans lequel la visualisation de la fraction est une barre de progression.

9. Procédé selon la revendication 8, dans lequel la barre de progression est formée concentriquement autour d'un point d'alignement dans la vue de navigation.

10. Procédé selon la revendication 9, dans lequel le point d'alignement se trouve sur une ligne droite entre le point d'entrée recommandé et la cible planifiée (50).

11. Procédé selon l'une quelconque des revendications précédentes, dans lequel le point d'entrée recommandé est de façon permanente passé du point d'entrée planifié (48) au point d'entrée actuel (66), lorsque la distance entre le point d'entrée planifié (48) et le point d'entrée actuel (66) satisfait à la condition prédéfinie.

12. Procédé selon l'une quelconque des revendications 1 à 10, dans lequel le point d'entrée recommandé est instantanément réinitialisé du point d'entrée actuel (66) au point d'entrée planifié (48), lorsque la distance entre le point d'entrée planifié (48) et le point d'entrée actuel (66) ne satisfait plus à la condition prédéfinie.

13. Processeur pour un système de navigation chirurgicale, le processeur configuré pour :
obtenir (40) des données de planification définissant un point d'entrée planifié (48) et une cible planifiée (50), dans lequel le point d'entrée planifié (48) et la cible planifiée (50) sont définis dans un système de coordonnées d'un modèle informatique d'au moins un élément anatomique ;
obtenir (42) des données de suivi décrivant une pose actuelle d'un instrument chirurgical (18) dans le système de coordonnées ;
déterminer (44), sur la base d'au moins les données de suivi, un point d'entrée actuel (66) se trouvant sur une trajectoire (56) ayant une relation spatiale fixe avec l'instrument chirurgical (18) ; et
**caractérisé en ce que** le processeur est configuré pour commander (46) un affichage d'une vue de navigation visualisant au moins un point d'entrée recommandé, dans lequel le point d'entrée recommandé est instantanément passé du point d'entrée planifié (48) au point d'entrée actuel (66), lorsqu'une distance entre le point d'entrée planifié (48) et le point d'entrée actuel (66) satisfait à une condition prédéfinie.

14. Support lisible par ordinateur comprenant un programme informatique, le programme informatique comprenant des instructions qui, lorsque le programme est exécuté par un processeur, amènent le processeur à mettre en œuvre le procédé selon l'une quelconque des revendications 1 à 12.

15. Signal de support de données transportant des informations qui représentent un programme informatique, le programme informatique comprenant des instructions qui, lorsque le programme est exécuté par un processeur, amènent le processeur à mettre en œuvre le procédé selon l'une quelconque des revendications 1 à 12.
